(19) Europäisches Patentamt
European Patent Office
Office européen des brevets

(11) Veröffentlichungsnummer: **0 053 823**
**B1**

(12) # EUROPÄISCHE PATENTSCHRIFT

(45) Veröffentlichungstag der Patentschrift: **10.04.85**

(21) Anmeldenummer: **81110167.4**

(22) Anmeldetag: **04.12.81**

(51) Int. Cl.[4]: **C 07 C 49/633,** C 07 C 49/653, C 07 B 57/00, C 07 C 43/305, C 07 C 45/42, C 07 C 41/50

(54) **Optisch reine Bicyclo(2.2.2)oct-5-en-2-one, Verfahren zu ihrer Herstellung und ihre Verwendung.**

(30) Priorität: **06.12.80 DE 3046106**

(43) Veröffentlichungstag der Anmeldung: **16.06.82 Patentblatt 82/24**

(45) Bekanntmachung des Hinweises auf die Patenterteilung: **10.04.85 Patentblatt 85/15**

(84) Benannte Vertragsstaaten: **AT BE CH DE FR GB IT LI LU NL SE**

(56) Entgegenhaltungen:
**HELVETICA CHIMICA ACTA, Band 62, Nr. 7, 1979 Basel S. ESCHER et al. "Neue Phellandren-Derivate aus dem Wurzelöl von Angelica archangelica L." Seiten 2061 bis 2072**
**HELVETICA CHIMICA ACTA, Band 63, Nr. 8 1980 Basel M. DEMUTH et al. "Photochemical High-yield Preparation of Tricyclo (3.3.0.0 2,8) octan-3-ones. Potential Synthons for Polycyclopentanoid Terpenes and Prostacyclin Analogs" Seiten 2434 bis 2439**
**TETRAHEDRON, Band 30, 1974 Oxford, New York, Braunschweig I. ALFARO et al. "Dihydroaromatic Compounds in the Diels-Alder Reaction - IV. Synthesis of Bicyclic Ketones" Seiten 559 bis 569**
**HOUBEN-WEYL "Methoden der organischen Chemie" 4. Auflage, Band IV, Teil 2 1955, GEORG THIEME VERLAG, Stuttgart Seiten 511 bis 529**

(73) Patentinhaber: **Studiengesellschaft Kohle mbH, Kaiser-Wilhelm-Platz 1, D-4330 Mülheim/Ruhr (DE)**

(72) Erfinder: **Demuth, Martin, Dr., Stiftstrasse 34-36, D-4330 Mühlheim/Ruhr (DE)**
Erfinder: **Schaffner, Kurt, Prof., Stiftstrasse 34-36, D-4330 Mülheim/Ruhr (DE)**

(74) Vertreter: **Werner, Hans-Karsten, Dr. et al, Deichmannhaus am Hauptbahnhof, D-5000 Köln 1 (DE)**

**EP 0 053 823 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft reine Enantiomere von Bicyclo [2.2.2]oct-5-en-2-onen, Verfahren zu ihrer Herstellung durch vollständige oder teilweise Ketalisierung und Trennung des Produktgemisches auf chromatographischem und destillativem Wege, sowie die Verwendung der enantiomeren Bicyclo[2.2.2]oct-5-en-2-one.

P.K. Freeman, D.M. Balls und D.J. Brown, J. Org. Chem. 33,2211 (1968) (siehe auch Literaturzitate 10 und 15 in S. Ranganathan, D. Ranganathan und A.K. Mehrotra, Syntheses 1977, 289) haben bereits die Synthese racemischer Gemische von Bicyclo[2.2.2] oct-5-en-2-onen ausgehend von substituierten Cyclohexa-1,4-dienen beschrieben. Ebenso haben I. Alfaro, W. Asthon, K.L. Rabone & N.A.J. Rogers, Tetrahedron 30, 559 (1974) und R.P. Gregson & R.N. Mirrington, Chem. Commun. 1973, 598 substituierte Bicyclo[2.2.2] oct-5-en-2-one beschrieben, wobei 2-Acetoxy- oder 2-Chloroacrylo-nitrile als En-Komponente eingesetzt wurden.

Die Anreicherung eines Enantiomeren ist bisher nur von K. Mislow & J.G. Berger, J. Am. Chem. Soc. 84, 1956 (1962) über die Umkristallisation von Ephedrin-Salzen beschrieben worden. Diese Anreicherung führte zu ca. 40% optischer Reinheit.

Die fotochemische Umwandlung von Racematen von Bicyclo[2.2.2]oct-5-en-2-onen zu racemischen Tricyclo[3.3.0.0$^{2,8}$]octan-3-onen bzw. zu Bicyclo[4.2.0] octenonen wurde von R.S. Givens, W.F. Oettle, R.L. Coffin & R.G. Carlson, J. Am. Chem. Soc. 93 3957 (1971) beschrieben. Auch in Contributed Paper No. 39 der IUPAC VIII/1980 in Seefeld, Österreich, wurde über die fotochemische Umwandlung von Racematen von Bicyclo[2.2.2]oct-5-en-2-onen durch C. Carter, S. Chandrasekhar, M. Demuth, K. Nakano & K. Schaffner, berichtet.

Die beschriebenen racemischen Bicyclo[2.2.2]oct-5-en-2-one enthalten nur H und eine Methylgruppe als Substituenten.

Aufgabe der vorliegenden Erfindung war es, die technisch wichtigen reinen Enantiomeren von Bicyclo[2.2.2]oct-5-en-2-onen zu erhalten, um von diesen Zugang zu den reinen Enantiomeren von Tricyclo[3.3.0.0$^{2,8}$]octanonen und Bicyclo[4.2.0]octenonen zu erhalten.

Die Erfindung betrifft daher Bicyclo[2.2.2]oct-5-en-2-one der Formeln Ia und Ib in Form reiner Enantiomerer

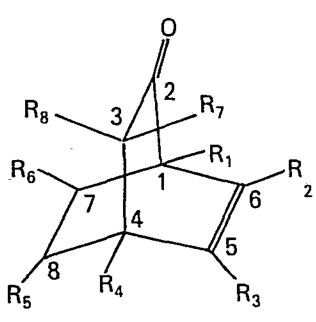

Ia

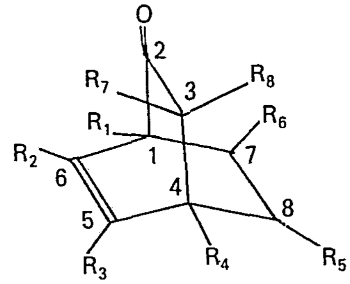

Ib

in denen $R_1$, $R_2$, $R_3$, $R_4$, $R_5$ und $R_6$ H,$C_1$-$C_8$-Alkyl-, $C_1$-$C_8$-Alkoxyl- und hydroxylierte und/oder carbonylierte $C_1$-$C_8$-Alkylgruppen sein können, die eine oder mehrere Doppelbindungen und/oder Dreifachbindungen enthalten können, ferner C1, Br und F und CN sein können sowie COOH und/oder deren Ester und

-C-R
||
O

(wobei R H,$C_1$-$C_8$-Alkykl-, $C_1$-$C_8$-Alkoxyl- und eine hydroxylierte und/oder carbonylierte $C_1$-$C_8$-Alkylgruppe sein kann, die eine oder mehrere Doppelbindungen und/oder Dreifachbindungen enthalten können und ferner die Carbonylgruppe acetalisiert oder ketalisiert sein kann), und $R_7$ und $R_8$ H,$C_1$-$C_8$-Alkyl-, $C_1$-$C_8$-Alkoxyl- und hydroxylierte und/oder carbonylierte $C_1$-$C_8$-Alkylgruppen sein können, die eine oder mehrere Doppelbindungen und/oder Dreifachbindungen enthalten können.

Die Erfindung betrifft weiterhin:

Ein Verfahren zur Herstellung reiner Enantiomerer von Bicyclo[2.2.2]oct-5-en-2-onen der Formeln Ia und Ib in der $R_1$, $R_2$, $R_3$, $R_4$, $R_5$, $R_6$, $R_7$ und $R_8$ die obengenannte Bedeutung haben, durch Diels-Alder-Addition von Cyclohexadienen der Formeln II und/oder III

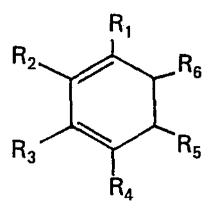

II

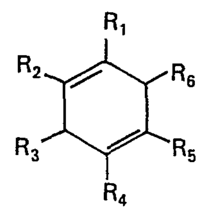

III

in denen $R_1$, $R_2$, $R_3$, $R_4$, $R_5$ und $R_6$ die obengenannte Bedeutung haben, mit Enen der Formel IV,

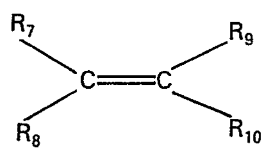

IV

in denen $R_7$ und $R_8$ die oben angegebene Bedeutung haben und $R_9$ und $R_{10}$ hydrolysierbare Substituenten, wie Halogene, -C≡N,

-O-C-$R_{11}$,
||
O

$-OR_{11}$, $NO_2$ oder Amine sind, wobei $R_{11}$ eine Alkylgruppe mit 1–4 C-Atomen ist, dadurch gekennzeichnet, dass man die durch Diels-Alder-Addition erhaltenen Racemate der Bicyclo[2.2.2]oct-5-en-2-one entweder vollständig ketalisiert mit reinen Enantiomeren von Diolen und die Ketale chromatographisch trennt oder bei nur teilweiser Ketalisierung das nicht oder weniger ketalisierte enantiomere Bicyclo[2.2.2]oct-5-en-2-on destillativ und/oder chromatographisch von dem/den ketalisierten Enantiomeren abtrennt und letztere ebenfalls chromatographisch trennt.

Ein Verfahren zur regioselektiven Herstellung von Bicyclo[2.2.2]oct-5-en-2-onen der Formel I, in der $R_1$, $R_2$, $R_3$, $R_4$, $R_5$, $R_6$, $R_7$ und $R_8$ die obengenannte Bedeutung haben, durch Diels-Alder-Addition von Cyclohexadienen der Formel II und III, in denen $R_1$, $R_2$, $R_3$, $R_4$, $R_5$ und $R_6$ die genante Bedeutung haben, mit Enen der Formel IV, in denen $R_7$ und $R_8$ die oben angegebene Bedeutung haben und $R_9$ und $R_{10}$ hydrolysierbare Substituenten, wie Halogene, -C≡N,

$$-O-\underset{\underset{O}{\|}}{C}-R_{11},$$

$-OR_{11}$, $NO_2$ oder Amine sind, wobei $R_{11}$ eine Alkylgruppe mit 1–4 C-Atomen ist, dadurch gekennzeichnet, dass man die Diels-Alder-Addition im Temperaturbereich von 60–120°C oder im Temperaturbereich von 140–200°C durchführt in Gegenwart von Polymerisationsinhibitoren, wie z.B. Hydrochinon.

Die Verwendung der Verbindungen gemäss Anspruch 1 zur Herstellung reiner Enantiomerer der Formel V durch sensibilisierte Fotoreaktion und von Verbindungen der Formel VI durch unsensibilisierte Fotoreaktion.

Bei den $C_1$-$C_8$-Alkyl-, $C_1$-$C_8$-Alkoxyl- und den hydroxylierten und carbonylierten $C_1$-$C_8$-Alkylgruppen handelt es sich um geradkettige und verzweigte Alkylgruppen.

Beispiele für Gruppen $C_1$-$C_8$ in $R_1$ bis $R_8$ und in R sind im einzelnen:

H, Methyl, Äthyl, iso- und n-Propyl, n-1-Butyl, n-2-Butyl, iso-Butyl, tert.-Butyl, von den $C_5$-$C_8$-Alkyl-, Alkoxyl- und hydroxylierten und carbonylierten Alkylgruppen sind die n-Alkyl-gruppen sowie die 2- und 3-Methyl-Alkylgruppen bevorzugt. Die Doppelbindungen, Dreifachbindungen, OH-Gruppen und C=O-Gruppen befinden sich in den $C_2$-$C_8$-Alkyl-, Alkoxyl- und hydroxylierten und carbonylierten Alkylgruppen bevorzugt in 1-, 2- und 3-Stellung.

Die Aufgabe wurde also dadurch gelöst, dass die racemischen Gemische der Bicyclo[2.2.2]oct-5-en-2-one, die durch Diels-Alder-Addition von Cyclohexadienen mit Enen gewonnen wurden, entweder vollständig ketalisiert wurden, wonach die Ketale chromatographisch getrennt wurden oder nur teilweise ketalisiert wurden. Das nicht ketalisierte Enantiomere wurde dann entweder destillativ abgetrennt und die gebildeten Ketale chromatographisch getrennt oder das Gemisch von nicht- und ketalisierten Enantiomeren ohne destillativen Schritt chromatographisch getrennt. Überraschend wurde gefunden, dass durch die chromatographische Trennung der Ketale, die durch Umsetzung der Bicyclo[2.2.2]oct-5-en-2-one mit optisch reinen Diolen erhalten wurden, beide optischen Antipoden der Bicyclo[2.2.2]oct-5-en-2-one in reiner Form gewonnen werden konnten. Als Diole eignen sich solche, die die OH-Gruppen in Nachbarstellung oder in 1,3-Stellung enthalten, wie z.B. Butan-2,3-diole, Pentan-2,4-diole, insbesondere aber L(+) und D(–)-Weinsäure und deren Ester.

Es wurde ferner überraschend gefunden, dass sich durch die Wahl der Substituenten am verwendeten Diol, die Wahl der Estergruppen der Weinsäureester und die Wahl der Substituenten am Bicyclo[2.2.2]oct-5-en-2-on und den Umsetzungsgrad die Ketalisierung in weitem Umfang steuern lässt.

So erhält man bei der Ketalisierung von racemischem 1-Methyl-bicyclo[2.2.2]oct-5-en-2-on mit L(+) Äthyltartrat Ketalisierung des (–)-1-Methyl-bicyclo[2.2.2]oct-5-en-2-ons bevorzugt im Verhältnis 1:0,4 gegenüber (+)-1-Methyl-bicyclo[2.2.2]oct-5-en-2-on.

Verwendet man als Ketalisierungsmittel L(+)-Diisopropyltartrat, so wird bei 50%iger Umsetzung praktisch nur das (–)-1-Methyl-bicyclo[2.2.2]oct-5-en-2-on ketalisiert, während das (+)-1-Methyl-bicyclo[2.2.2]oct-5-en-2-on nahezu unverändert bleibt und abdestilliert werden kann.

Weitere bevorzugte Ester sind Phenyl- und Methylester.

Zur Herstellung der racemischen Bicyclo[2.2.2]oct-5-en-2-one eignen sich 1,4-Cyclohexadiene und 1,3-Cyclohexadiene, in denen die Substituenten $R_1$, $R_2$, $R_3$, $R_4$, $R_5$ und $R_6$ H,$C_1$-$C_8$-Alkyl-, $C_1$-$C_8$-Alkoxyl- und hydroxylierte und carbonylierte $C_1$-$C_8$-Alkylgruppen sein können, die eine oder mehrere Doppelbindungen und/oder Dreifachbindungen enthalten können, ferner Cl, Br, F und CN sein können, sowie COOH und/oder deren Ester und/oder COR (wobei RH,$C_1$-$C_8$-Alkyl-, $C_1$-$C_8$-Alkoxyl- und hydroxylierte und/oder carbonylierte $C_1$-$C_8$-Alkylgruppen sein kann, die eine oder mehrere Doppelbindungen und/oder Dreifachbindungen enthalten können und ferner die Carbonylgruppe acetalisiert bzw. ketalisiert sein kann). Als En-Komponente wird das Olefin gemäss Formel IV verwendet, in der $R_7$ und $R_8$ die oben angegebene Bedeutung haben und $R_9$ und $R_{10}$ hydrolysierbare Substituenten, wie Halogene, -C≡N,

$$-O-\underset{\underset{O}{\|}}{C}-R_{11}$$

oder $-OR_{11}$, $NO_2$ oder Amine sind, wobei $R_{11}$ eine Alkylgruppe mit 1–4 C-Atomen ist.

Die Diels-Alder-Addition kann unter üblichen Bedingungen erfolgen, es wurde jedoch überraschend gefunden, dass bei Einhaltung bestimmter Temperaturbereiche hohe Regioselektivität der Addition erzielt werden kann. Führt man z.B. in Gegenwart von Polymerisationsinhibitoren, wie z.B. Hydrochinon, die Addition von 1-Methylcyclohexadien und 2-Chloracrylnitril als En-Komponente im Temperaturbereich von 60–120°C, vorzugsweise bei 110°C, durch, so erhält man ein 5:1-Gemisch der Isomeren 7 und 8, arbeitet man jedoch im Temperaturbereich von 140–200°C, vorzugsweise bei 150°C, so erhält man das Isomere 8 in reiner Form (7 = Formel Ia mir $R_1$ = Methyl; 8 = Formel Ib mit $R_1$ = Methyl).

Die Ketalisierung der Bicyclo[2.2.2]oct-5-en-2-one mit Diolen erfolgt in üblicher Weise in Gegenwart von Säuren. Die Umsetzung kann sowohl in Lösungsmitteln als auch ohne Lösungsmittel erfolgen, wobei man vorteilhaft das sich während der Reaktion bildende Wasser laufend dem Reaktionsgemisch entzieht. Die Trennung der Ketale erfolgt säulen-chromatographisch, kann jedoch auch durch andere chromatographische Methoden durchgeführt werden. Nach erfolgter Trennung kann man durch übliche hydrolytische Spaltung der Ketale die reinen Enantiomeren der Bicyclo[2.2.2]oct-5-en-2-one erhalten.

Werden die reinen Enantiomeren der Bicyclo[2.2.2]oct-5-en-2-one in Gegenwart von Sensibilisatoren, wie z.B. Acetophenon, Aceton, Benzophenon oder Benzol, bestrahlt, insbesondere in einem Wellenlängenbereich von 254–400 nm, so erhält man überraschend in selektiver Weise das entsprechende reine Enantiomere des Tricyclo[3.3.0.0$^{2,8}$]octan-3-ons der Formel V, bestrahlt man ein reines Enantiomeres des Bicyclo[2.2.2]oct-5-en-2-ons ohne Zusatz von Sensibilisatoren, so wird überraschend ein reines enantiomeres Bicyclo[4.2.0]octenon der Formel VI erhalten. Die Verwendung der reinen Enantiomeren von Bicyclo[2.2.2]oct-5-en-2-onen erlaubt daher erstmalig, diese technisch wertvollen Produkte in optisch reiner Form selektiv synthetisch zu gewinnen. Die Erfindung wird in den folgenden Beispielen näher erläutert.

Beispiel 1

Ein Gemisch von 1-[Pentenyl-3]-1,4-cyclohexadien und 2-Chloracrylnitril wurde in Gegenwart von Hydrochinon als Polymerisationsinhibitor zehn Stunden auf 120°C erhitzt. Das Rohprodukt wurde bei 71–73°C/0,13 mbar destilliert und das Destillat in einer wässrigen Lösung von Kalium-Hydroxid in Dimethylsulfoxid bei 120° während 30 Minuten hydrolysiert. Das Gemisch wurde dann auf Eis geschüttet. Extraktion mit Pentan ergab ein racemisches Gemisch von 4-[Pentenyl-3]-bicyclo[2.2.2]oct-5-en-2-on in einer Ausbeute von 58%.

Analyse in %:

| | | | |
|---|---|---|---|
| ber.: | C: 82,11 | H: 9,47 | O: 8,42, |
| gef.: | C: 82,1 | H: 9,5 | O: 8,3. |

Beispiel 2

Ein Gemisch von 1-Methyl-1,4-cyclohexadien und 2-Chlor-acrylnitril wurde in Gegenwart von Hydrochinon als Polymerisationsinhibitor 14 Stunden auf 110°C erhitzt. Das Rohprodukt wurde bei 61–65°C/0,13 mbar destilliert und das Destillat in einer wässrigen Lösung von Kalium-Hydroxid in Dimethylsulfoxid bei 120° C während 30 Minuten hydrolysiert. Das Gemisch wurde anschliessend auf Eis gegossen. Extraktion mit Pentan und chromatographische Trennung auf einer Silikagel-Säule führte in 50–55%iger Ausbeute zu einem 5:1-Gemisch von 4-Methyl-bicyclo[2.2.2]oct-5-en-2-on und 1-Methyl-bicyclo[2.2.2]oct-5-en-2-on.

Analyse in %:

| | | | |
|---|---|---|---|
| ber. | C: 79,41 | H: 8,82 | O: 11,76, |
| gef. | C: 79,3 | H: 8,9 | O: 11,7. |

Wurde die gleiche Umsetzung bei 150°C durchgeführt, so wurde ausschliesslich das 1-Methyl-Derivat in 54%iger Ausbeute erhalten. Beide Produkte lagen in racemischer Form vor.

Beispiel 3

5,5 g von 1-Methyl-bicyclo[2.2.2]oct-5-en-2-on wurden zusammen mit 12 g L(+)-Äthyltartrat und 350 mg p-Toluolsulfonsäure in 40 ml Benzol am Rückfluss und Wasserabscheider gekocht. Bei einer Umsetzung von 50% wurde das Reaktionsprodukt aufgearbeitet durch Extrahieren der Benzollösung mit Wasser. Nach Eindampfen der organischen Phase erhielt man 12 g Rohmaterial, das an Silikagel (0,043–0,063 mm, 400 g) chromatographiert wurde mit Toluol als Lösungsmittel. Die anfallenden Mischfraktionen wurden wiederholt chromatographiert. Es wurden 2,75 g Ketal des (–)-1-Methyl-bicyclo[2.2.2]oct-5-en-2-ons und 1,2 g Ketal des (+)-1-Methyl-bicyclo[2.2.2]oct-5-en-2-ons erhalten. Beide Enantiomere konnten nach üblicher Hydrolyse der Ketale in reiner Form gewonnen werden. Nicht umgesetztes Einsatzprodukt wurde annähernd quantitativ zurückgewonnen.

Beispiel 4

6 g von 1-Methyl-bicyclo[2.2.2]oct-5-en-2-on wurden zusammen mit 18 g L(+)-Diisopropyltartrat und 400 mg p-Toluolsulfonsäure in 50 ml Benzol am Rückfluss und Wasserabscheider gekocht.

Bei einer Umsetzung von 50% wurde das Reaktionsprodukt aufgearbeitet durch Extrahieren der Benzollösung mit Wasser. Nach Eindampfen der organischen Phase wurden 14 g Rohmaterial erhalten, das bei 60°C/1,3 mbar destilliert wurde. Das überdestillierte Produkt bestand nahezu ausschliesslich aus (+)-1-Methyl-bicyclo[2.2.2]oct-5-en-2-on. Bei 110°C/1,3 mbar destillierte das Ketal über, das aus praktisch reinem Ketal des (–)-1-Methyl-bicyclo[2.2.2]oct-5-en-2-ons bestand.

Beispiel 5

3,5 g racemisches 4-Methyl-bicyclo[2.2.2]oct-5-en-2-on wurden mit 14 g L(+)-Butyltartrat und 1

g stark-saurem Ionenaustauscher bei Raumtemperatur in 50 ml Cyclohexan 48 Stunden geschüttelt. Nach dieser Zeit hatten sich 85% des Einsatzproduktes umgesetzt. Das Gemisch wurde darauf filtriert und das Filtrat mit Wasser gut gewaschen. Der Rückstand der organischen Phase wurde mit Toluol an Silikagel-Säulen chromatographiert. Nach zwei Reinigungsdurchgängen erhielt man etwa gleiche Mengen der Ketale der reinen Enantiomeren. Nicht umgesetztes Einsatzprodukt, wurde nahezu quantitativ zurückgewonnen.

Beispiel 6

2 g racemisches 1-Butyl-bicyclo[2.2.2]oct-5-en-2-on wurden mit 7 g D(-)-Weinsäure in 25 ml Methanol und in Gegenwart von Molekular-Sieb während 36 Stunden auf 40°C erwärmt. Die Reaktion verlief annähernd quantitativ. Die chromatographische Trennung des Rohproduktes mit Äthylacetat/Methanol (2:1) auf Silikagel führte zu den Ketalen der reinen Enantiomeren.

Beispiel 7

Eine Aceton-Lösung (1%ig, 1 g) von (-)-1-Methyl-bicyclo[2.2.2]oct-5-en-2-on wurde mit Argon entgast und in einem wassergekühlten Quarzgefäss mit einer 3 000 Angström-Lampe belichtet. Nach 72 Stunden wurde eine Umsetzung von 96 % gemessen. Das Aceton wurde abdestilliert und der Rückstand an 15 g Silikagel chromatographiert. Mit Benzol erhielt man nicht umgesetztes Einsatzprodukt und mit Benzol/Äther (9:1) 0,86 g (+)-8-Methyl-tricyclo[3.3.0.0$^{2,8}$]octan-3-on.

(Analyse:

| | | | |
|---|---|---|---|
| ber. | C: 79,41 | H: 8,82 | O: 11,76, |
| gef. | C: 79,5 | H: 8,8 | O: 11,6.) |

Beispiel 8

Eine 4%ige Lösung von (+)-1-Butyl-bicyclo[2.2.2]oct-5-en-2-on (1,5 g) in Cyclohexan wurde in einer Pyrex-Apparatur unter Argon und in Gegenwart von 200 mg Acetophenon mit einer 250 W Quecksilber-Mitteldruck-Lampe belichtet. Nach 50 Stunden war eine Umsetzung von 98% erreicht. Die Lösung wurde eingedampft und der Rückstand an 100 g Silikagel (70–230 mesh) mit Toluol zur Entfernung des Acetophenons und Toluol/1% Diäthyläther chromatographiert. Es wurde reines (-)-8-Butyl-tricyclo[3.3.0.0$^{2,8}$]octan-3-on erhalten.

(Analyse:

| | | | |
|---|---|---|---|
| ber. | C: 80,9 | H: 10,11 | O: 8,99 |
| gef. | C: 80,9 | H: 10,0 | O: 8,8.) |

Beispiel 9

Eine 4%ige Lösung von (+)-Bicyclo[2.2.2]oct-5-en-2-on wurde in Cyclohexan gelöst und in einer Pyrex-Apparatur unter Argon mit einer 250 W Quecksilber-Mitteldruck-Lampe belichtet. Nach 50 Stunden hatte sich das Einsatzprodukt quantitativ umgesetzt. Die Lösung wurde eingedampft und der Rückstand an 100 g Silikagel mit Toluol/1% Diäthyläther chromatographiert. Es wurde reines (-)-Bicyclo[4.2.0]oct-3-en-8-on erhalten.

(Analyse in %:

| | | | |
|---|---|---|---|
| ber. | C: 78,69 | H: 8,19 | O: 13,11 |
| gef. | C: 78,7 | H: 8,2 | O: 12,9.) |

Spektroskopische Daten

1-Methyl-bicyclo[2.2.2]oct-5-en-2-on

NMR 6,38 (m, 1H) 5,72 (m, 1H) 3,2/1,3 (7H) 1,2 (s, 3H) Lösm. $CDCl_3$
IR 3120, 3020, 2950, 1725, 1620, 1450, 1410, 1095 $cm^{-1}$ (ohne Lösm.)
Drehwert $[\alpha]_D$ + oder −495° ± 5% Lösm. Chloroform, 23°C

4-Methyl-bicyclo[2.2.2]oct-5-en-2-on

NMR 5,95 (m, 2H) 2,9/1,3 (7H) 1,27 (s, 3H) Lösm. $CDCl_3$
IR 3110, 3020, 2930, 1720, 1650, 1460, 1360, 1250, 1190, 1100, (ohne Lösm.)
Drehwert $[\alpha]_D$ + oder −515° ± 5% Lösm. $CHCl_3$, 23°C

Ketal aus L(+)-Äthyltartrat und (+)-1-Methyl-bicyclo[2.2.2]oct-5-en-2-on

NMR 6,35 (m, 2H) 4,7/4,6 (dd, 2H) 4,3/4,28 (je g, je 2H) 3,1/1,5 (7H) 1,35/1,38 (je t, je 3H) 1,25 (s, 3H) Lösm. $CDCl_3$
IR 3000, 1755, 1750, 1460, 1435, 1370, 1340, 1265, 1210, 1020
$[\alpha]_D$ −85° ± 5% Lösm. $CHCl_3$, 23°C

Ketal aus L(+)-Äthyltartrat und (-)-1-Methyl-bicyclo[2.2.2]oct-5-en-2-on

NMR 6,3 (m, 2H) 4,8/4,6 (dd, 2H) 4,29/4,26 (je q, je 2H) 3,1/1,5 (7H) 1,35/1,30 (je t, je 3H) 1,22 (s, 3H) Lösm. $CDCl_3$
IR 3000, 1760, 1755, 1460, 1440, 1370, 1270, 1130, 1025 $cm^{-1}$ ohne Lösm.
$[\alpha_{-D}$ +45° ± 5% Lösm. $CHCl_3$, 23°C

**Patentansprüche**

1. Bicyclo[2.2.2]oct-5-en-2-one der Formeln Ia und Ib in Form reiner Enantiomerer

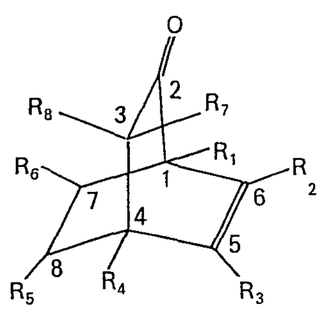

Ia

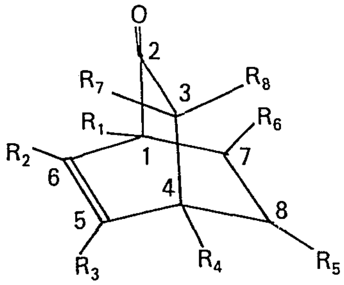

Ib

in denen $R_1$, $R_2$, $R_3$, $R_4$, $R_5$ und $R_6$

a) H,$C_1$-$C_8$-Alkyl-, $C_1$-$C_8$-Alkoxyl- und hydroxylierte und/oder carbonylierte $C_1$-$C_8$-Alkylgruppen sein können, die eine oder mehrere Doppelbindungen und/oder Dreifachbindungen enthalten können,

b) Cl, Br und F und CN sein können sowie

c) COOH und/oder deren Ester und

d) -C-R
||
O

(wobei R H,$C_1$-$C_8$-Alkyl-, $C_1$-$C_8$-Alkoxyl- und eine hydroxylierte und/oder carbonylierte $C_1$-$C_8$-Alkylgruppe sein kann, die eine oder mehrere Doppelbindungen und/oder Dreifachbindungen enthalten können, und ferner die Carbonylgruppe acetalisiert oder ketalisiert sein kann), und $R_7$ und $R_8$ H,$C_1$-$C_8$-Alkyl-, $C_1$-$C_8$-Alkoxyl- und hydroxylierte und/oder carbonylierte $C_1$-$C_8$-Alkylgruppen sein können, die eine oder mehrere Doppelbindungen und/oder Dreifachbindungen enthalten können.

2. Verfahren zur Herstellung reiner Enantiomerer von Bicyclo[2.2.2]oct-5-en-2-onen der Formeln Ia und Ib, gemäss Anspruch 1, in der $R_1$, $R_2$, $R_3$, $R_4$, $R_5$, $R_6$, $R_7$ und $R_8$ die im Anspruch 1 genannte Bedeutung haben, durch Diels-Alder Addition von Cyclohexadienen der Formeln II und III

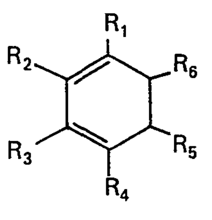

II

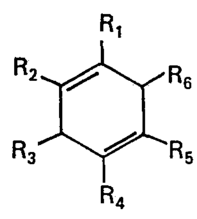

III

in denen $R_1$, $R_2$, $R_3$, $R_4$, $R_5$ und $R_6$ die im Anspruch genannte Bedeutung haben, mit Enen der Formel IV

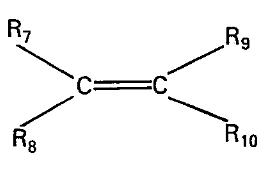

IV

in denen $R_7$ und $R_8$ die im Anspruch 1 angegebene Bedeutung haben und $R_9$ und $R_{10}$ hydrolysierbare Substituenten, wie Halogene, -C≡N,

-O-C-$R_{11}$
||
O

-$OR_{11}$, $NO_2$ oder Amine sind, wobei $R_{11}$ eine Alkylgruppe mit 1-4 C-Atomen ist, dadurch gekennzeichnet, dass man die durch Diels-Alder-Addition erhaltenen Racemate der Bicyclo[2.2.2]oct-5-en-2-one entweder vollständig ketalisiert mit reinen Enantiomeren von Diolen, die die OH-Gruppen in Nachbarstellung oder in 1,3-Stellung enthalten, und die Ketale chromatographisch trennt, oder dass man die Ketalisierung nur teilweise durchführt und das nicht oder weniger ketalisierte enantiomere Bicyclo[2.2.2]oct-5-en-2-on destillativ und/oder chromatographisch von dem/den ketalisierten Enantiomeren abtrennt und letztere ebenfalls chromatographisch trennt.

3. Verfahren nach Anspruch 2, dadurch gekennzeichnet, dass man als Diol L(+) oder D(–)-Weinsäure oder deren Ester verwendet.

4. Verfahren zur regioselektiven Herstellung von Racematen von Bicyclo[2.2.2]oct-5-en-2-onen der Formeln Ia und Ib in denen $R_1$, $R_{2,\,3}$, $R_4$, $R_5$, $R_6$, $R_7$ und $R_8$ die im Anspruch 1 genannte Bedeutung haben, durch Diels-Alder-Addition von Cyclohexadienen der Formel II und/oder III, in denen $R_1$, $R_2$, $R_3$, $R_4$, $R_5$ und $R_6$ die im Anspruch 1 genannte Bedeutung haben, mit Enen der Formel IV, in denen $R_7$ und $R_8$ die im Anspruch 1 angegebene Bedeutung haben und $R_9$ und $R_{10}$ hydrolysierbare Substituenten, wie Halogene, -C≡N,

-O-C-$R_{11}$,
||
O

-$OR_{11}$, $NO_2$ oder Amine sind, wobei $R_{11}$ eine Alkylgruppe mit 1-4 C-Atomen ist, dadurch gekennzeichnet, dass man die Diels-Alder-Addition im Temperaturbereich von 60-120°C oder im Temperaturbereich 140-200°C durchführt in Gegenwart von Polymerisationsinhibitoren, wie z.B. Hydrochinon.

5. Verwendung der Verbindungen gemäss Anspruch 1 zur Herstellung reiner Enantiomerer der Formel V durch sensibilisierte Fotoreaktion und von Verbindungen der Formel VI durch unsensibilisierte Fotoreaktion.

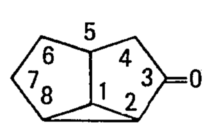

V

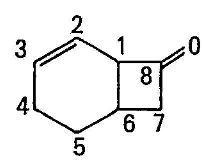

VI

**Claims**

1. Bicyclo[2.2.2.]oct-5-ene-2-ones having the formulae Ia and Ib, in the forms of pure enantiomers,

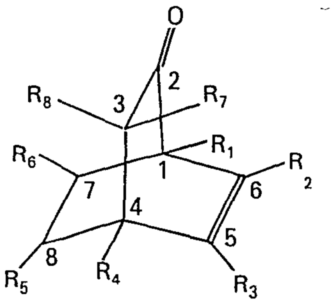

Ia

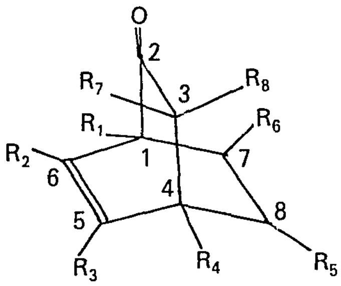

Ib

wherein

$R_1$, $R_2$, $R_3$, $R_4$, $R_5$ and $R_6$ may be
a) H, $C_1$-$C_8$-alkyl, $C_1$-$C_8$alkoxyl and hydroxylated and/or carbonylated $C_1$-$C_8$-alkyl groups which may contain one or several double bonds and/or triple bonds,
b) C1, Br and F and CN as well as
c) COOH and/or the esters thereof and

d) 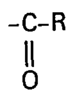

(wherein R may be H, $C_1$-$C_8$-alkyl, $C_1$-$C_8$-alkoxyl and a hydroxylated and/or carbonylated $C_1$-$C_8$-alkyl group which may contain one or several double bonds and/or triple bonds and further the carbonyl group may be acetalized or ketalized), and
$R_7$ and $R_8$ may be H, $C_1$-$C_8$-alkyl, $C_1$-$C_8$-alkoxyl and hydroxylated and/or carbonylated $C_1$-$C_8$-alkyl groups which may contain one or several double bonds and/or triple bonds.

2. A process for preparing pure enantiomers of bicyclo[2.2.2.]oct-5-ene-2-ones having the formulae Ia and Ib according to claim 1, wherein $R_1$, $R_2$, $R_3$, $R_4$, $R_5$, $R_6$, $R_7$ and $R_8$ are as defined in claim 1 by Diels-Alder addition of cyclohexadienes having the formulae II and III

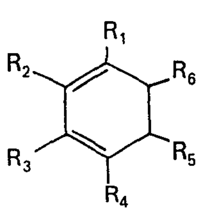

II

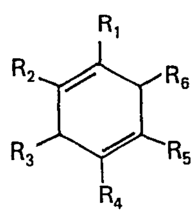

III

wherein
$R_1$, $R_2$, $R_3$, $R_4$, $R_5$ and $R_6$ are as defined in claim 1 to enes having the formula IV

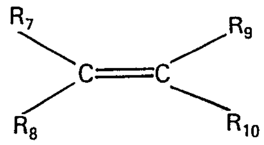

IV

wherein
$R_7$ and $R_8$ are as defined in claim 1 and
$R_9$ and $R_{10}$ are hydrolyzable substituents such as halogens, -C≡N,

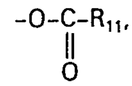

$-OR_{11}$, $NO_2$ or amines, wherein $R_{11}$ is an alkyl group having from 1 to 4 carbon atoms, characterized in that the racemates obtained by the Diels-Alder addition of the bicyclo[2.2.2.]oct-5-ene-2-ones are either completely ketalized with pure enantiomers of diols containing the OH groups in the vicinal position or in the 1,3-position and the ketals are chromatographically separated, or that the ketalization is only carried out to occur partially and the enantiomeric bicyclo-[2.2.2.]oct-5-ene-2-one having been not or less ketalized is separated by means of distillation or chromatography from the ketalized enantiomer(s) and the latter are also chromatographically separated.

3. The process according to claim 2, characterized in that L(+)- or D(−)-tartaric acid or the esters thereof are used as the diol.

4. A process for regioselectively preparing racemates of bicyclo[2.2.2.]oct-5-ene-2-ones having the formulae Ia and Ib according to claim 1, wherein $R_1$, $R_2$, $R_3$, $R_4$, $R_5$, $R_6$, $R_7$ and $R_8$ are as defined in claim 1, by Diels-Alder addition of cyclohexadienes having the formulae II and/or III, wherein $R_1$, $R_2$, $R_3$, $R_4$, $R_5$ and $R_6$ are as defined in claim 1, to enes having the formula IV, wherein $R_7$ and $R_8$ are as defined in claim 1 and $R_9$ and $R_{10}$ are hydrolyzable substituents such as halogens, -C≡N,

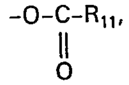

$-OR_{11}$, $NO_2$ or amines, wherein $R_{11}$ is an alkyl group having from 1 to 4 carbon atoms, characterized in that the Diels-Alder addition is carried out in the temperature range of from 60°C to 120°C or in the temperature range of from 140°C to 200°C in the presence of polymerization inhibitors such as, e.g., hydroquinone.

5. Use of the compounds according to claim 1 for the preparation of pure enantiomers represented by the formula V by a sensitized photoreaction and of compounds represented by the formula VI by an unsensitized photoreaction.

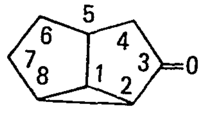

V

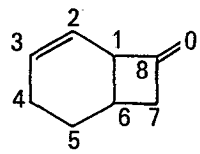

VI

## Revendications

1. Bicyclo[2.2.2.]octène-5 ones-2, sous forme d'énantiomères purs, répondant aux formules Ia et Ib:

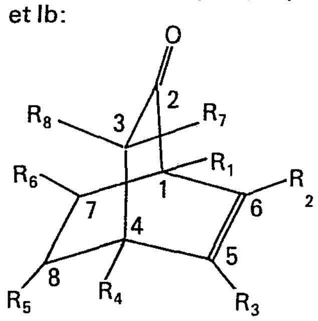

Ia

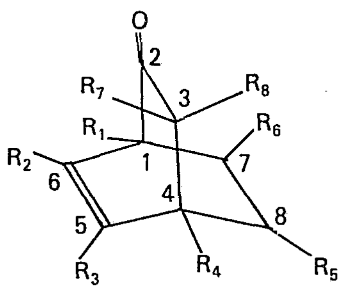

Ib

dans lesquelles $R_1$, $R_2$, $R_3$, $R_4$, $R_5$ et $R_6$ peuvent être:

(a) H, un groupe alkyle en $C_1$ à $C_8$, alcoxyle en $C_1$ à $C_8$ et un groupe alkyle en $C_1$ à $C_8$, hydroxylé et/ou carbonylé, pouvant contenir une ou plusieurs doubles liaisons et/ou triples liaisons,
(b) C1, Br, F et CN,
(c) COOH et/ou ses esters, et

(d) -C-R
‖
O

(où R peut représenter H, un groupe alkyle en $C_1$ à $C_8$, alcoxyle en $C_1$ à $C_8$ et un groupe alkyle en $C_1$ à $C_8$ hydroxylé et/ou carbonylé, pouvant contenir une ou plusieurs doubles liaisons et/ou triples liaisons, le groupe carbonyle pouvant en outre être acétalisé ou cétalisé), et $R_7$ et $R_8$ pouvant représenter H, un groupe alkyle en $C_1$ à $C_8$, alcoxyle en $C_1$ à $C_8$ et un groupe alkyle en $C_1$ à $C_8$ hydroxylé et/ou carbonylé, pouvant contenir une ou plusieurs doubles liaisons et/ou triples liaisons.

2. Procédé de préparation d'énantiomères purs de bicyclo (2.2.2) octène-5 ones-2 répondant aux formules Ia et Ib, selon la revendication 1, dans lesquelles, $R_1$, $R_2$, $R_3$, $R_4$, $R_5$, $R_6$, $R_7$ et $R_8$ ont les sens indiqués à la revendication 1, par addition de Diels-Alder de cyclohexadiènes répondant aux formules II et III:

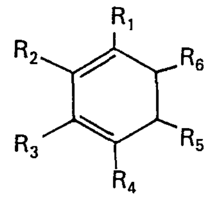

II

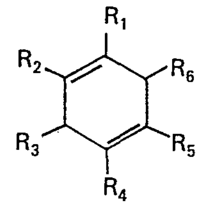

III

(dans lesquelles $R_1$, $R_2$, $R_3$, $R_4$, $R_5$ et $R_6$ ont le sens indiqué à revendication 1) avec des ènes de formule IV:

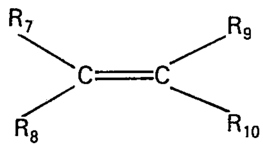

IV

(dans laquelle $R_7$ et $R_8$ ont le sens indiqué à la revendication 1, et $R_9$ et $R_{10}$ sont des substituants hydrolysables, comme des halogènes, -C≡N,

-O-C-$R_{11}$,
‖
O

-$OR_{11}$, $NO_2$ ou des amines ($R_{11}$ étant un groupe alkyle ayant 1 à 4 atomes de carbone), procédé caractérisé en ce que l'on cétalise entièrement, à l'aide d'énantiomères purs de diols contenant les groupes OH en positions voisines ou en 1,3, les racémates des bicyclo (2.2.2) octène-5 ones-2 obtenus par addition de Diels-Alder, et l'on sépare les cétals par chromatographie, ou bien en ce que l'on n'effectue que partiellement la cétalisation, on sépare; par distillation et/ou par chromatographie, la bicyclo (2.2.2) octène-5 ones-2 énantiomère non ou moins cétalisée du ou des énantiomères cétalisé(s) et l'on sépare également par chromatographie ce ou ces derniers.

3. Procédé selon la revendication 2, caractérisé en ce qu'on utilise comme diol l'acid L(+) ou D(−) tartrique ou ses esters.

4. Procédé de préparation régiosélective de racémates de bicyclo (2.2.2) octène-5 ones-2 répondant aux formules Ia et Ib (dans lesquelles $R_1$, $R_2$, $R_3$, $R_4$, $R_5$, $R_6$, $R_7$ et $R_8$ ont le sens indiqué à la revendication 1), par addition de Diels-Alder de cyclohexadiènes répondant aux formules II et/ou III (dans lesquelles $R_1$, $R_2$, $R_3$, $R_4$, $R_5$ et $R_6$ ont le sens indiqué à la revendication 1) avec des ènes de formule IV (dans laquelle $R_7$ et $R_8$ ont le sens indiqué à la revendication 1, et $R_9$ et $R_{10}$ sont des substituants hydrolysables, comme des halogènes, -C≡N,

-O-C-$R_{11}$ -$OR_{11}$, $NO_2$
‖
O

ou des amines, $R_{11}$ étant un groupe alkyle comportant 1 à 4 atomes de carbone), procédé caractérisé en ce qu'on effectue l'addition de Diels-Alder dans l'intervalle de températures de 60 à

120°C ou dans l'intervalle de températures de 140 à 200°C, en présence d'inhibiteurs de polymérisation, comme par exemple l'hydroquinone.

5. Utilisation de composés selon la revendication 1 pour préparer des énantiomères purs de formule V par photoréaction sensibilisée ou de composés de formule VI par photoréaction non sensibilisée.

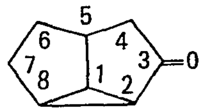

V

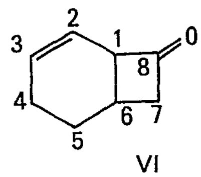

VI